(19) Europäisches Patentamt
European Patent Office
Office européen des brevets

(11) Veröffentlichungsnummer: **0 529 424 A2**

(12) **EUROPÄISCHE PATENTANMELDUNG**

(21) Anmeldenummer: **92113794.9**

(22) Anmeldetag: **13.08.92**

(51) Int. Cl.5: **C12N 11/08**, C12P 41/00, C12N 9/20

(30) Priorität: **30.08.91 AT 1715/91**

(43) Veröffentlichungstag der Anmeldung:
**03.03.93 Patentblatt 93/09**

(84) Benannte Vertragsstaaten:
**AT BE CH DE DK ES FR GB IE IT LI NL SE**

(71) Anmelder: **Chemie Linz Gesellschaft m.b.H.**
**St.Peter-Strasse 25**
**A-4021 Linz(AT)**

(72) Erfinder: **Faber, Kurt, Dr.**
**Hafnerriegel 64**
**A-8010 Graz(AT)**
Erfinder: **Berger, Brigitte, Dipl.-Ing.**

**A-9761 Greifenburg 192(AT)**

(74) Vertreter: **Kunz, Ekkehard, Dr.**
**Chemie Linz GesmbH., Patentwesen, St.**
**Peter Strasse 25**
**A-4021 Linz (AT)**

(54) **Verfahren zur Erhöhung der Enantioselektivität einer Candida Lipase bei der Veresterung chiraler Alkohole und eine immobilisierte Candida Lipase.**

(57) Lipase aus einem Mikroorganismus der Gattung Candida, die an epsilon-Aminogruppen des Lysins mit geöffneten Epoxidgruppen eines epoxid-aktivierten makroskopischen Trägers durch N-Alkylierung kovalent gebunden und damit immobilisiert ist, ein Verfahren zu deren Herstellung, deren Verwendung in einem Verfahren zur enantioselektiven Veresterung von Enatiomerengemischen eines Alkohols, der mindestens ein Asymmetriezentrum im Molekül enthält, mit Hilfe eines Enolesters, sowie ein Verfahren zur enantioselektiven Umesterung eines Enantiomerengemisches eines Alkohols, der mindestens ein Asymmetriezentrum im Molekül enthält, mit Hilfe der immobilisierten Lipase in Gegenwart eines Enolesters.

Die Erfindung betrifft ein Verfahren zur Erhöhung der Enantioselektivität, Aktivität und Stabilität einer Lipase aus einem Mikroorganismus der Gattung Candida in einem enzymatischen Umesterungsverfahren, in dem ein chiraler Alkohol, der mindestens ein Asymmetriezentrum im Molekül enthält, mit Hilfe eines Enolesters enantioselektiv verestert wird, eine immobilisierte Candida Lipase und ein Verfahren zu deren Herstellung.

Chirale, enantiomerenreine Alkohole sind für verschiedenste Anwendungszwecke, etwa zur Synthese pharmazeutischer Wirkstoffe oder Agrarchemikalien von Bedeutung. Ihre Herstellung kann z.B. durch enantioselektive Umesterung erfolgen, wobei ein Enantiomerengemisch eines chiralen Alkohols in Gegenwart eines Carbonsäureesters mit Hilfe einer Hydrolase umgesetzt wird. Da Hydrolasen im allgemeinen sowohl die Hin-, als auch die Rückreaktion katalysieren, ensteht das gewünschte Endprodukt bei solchen Umsetzungen häufig nur sehr langsam und in ungenügender optischer Reinheit. In M.Degueil-Castaing et al, Tetrahedron Letters, Vol 28, 9 (1987), 953-954 wird daher vorgeschlagen, als Carbonsäureester einen Enolester einzusetzen, da eine Rückreaktion dann nicht mehr erfolgen kann. Wie jedoch aus Zakrzewska et al, Acta Med. Pol., 29, (1988), 1-2, Seite 44 hervorgeht, können die Aldehyde, die bei der Umesterung aus dem Enolester entstehen, durch Reaktion mit endständig funktionellen Gruppen von Aminosäuren des Enzyms das Enzym desaktivieren. In EP-A-0 321 918 ist ein Verfahren zur enzymatischen Racematspaltung von Enantiomergemischen eines racemischen Alkohols mit oder in einem Vinylester mit Hilfe einer Lipase beschrieben, bei dem der freigesetzte Aldehyd die Lipase nicht desaktivieren soll. Es hat sich aber herausgestellt, daß beim wiederholten Einsatz einer Candida Lipase in einem solchen Verfahren sowohl deren Aktivität als auch deren Selektivität sehr rasch stark absinkt.

In C.J.Gray et al, Enzyme Microb. Technol., Vol 12 (1990), Seiten 800 bis 807, ist geoffenbart, daß die Stabilität einer Candida cylindracea Lipase durch verschiedene Immobiliserungstechniken für einem wiederholten Gebrauch erhöht werden kann. Umesterungen unter Einsatz von Enolester sind dort aber nicht erwähnt.

Es wurde nun unerwarteterweise gefunden, daß sich sowohl die Stabilität gegen Aldehyde, als auch die Aktivität und ungewöhnlicherweise auch die Selektivität einer Candida Lipase erhöhen, wenn die Lipase vor ihrem Einsatz in einem Umesterungsverfahren unter Verwendung von Enolestern durch Umsetzung von epsilon-Aminogruppen des Lysins der Lipase mit Epoxidgruppen eines epoxid-aktivierten makroporösen Trägers, wobei eine N-Alkylierung stattfindet, immobilisiert wird. Durch diese Immobilisierung wird sowohl die Beständigkeit der Lipase gegenüber Aldehyden als auch ihre Aktivität und Substratselektivität im Vergleich zu einer nicht immobilisierten Lipase erhöht, wobei die Substratselektivität bei wiederholtem Einsatz nicht absinkt, sondern völlig konstant bleibt.

Gegenstand der Erfindung ist daher eine Lipase aus einem Mikroorganismus der Gattung Candida, die dadurch gekennzeichnet ist, daß epsilon-Aminogruppen des Lysins in der Lipase durch N-Alkylierung kovalent über geöffnete Epoxidgruppen eines epoxid-aktivierten makroporösen Trägers gebunden sind, wodurch die Lipase immobilisiert wird.

Unter Lipase aus einem Mikroorganismus der Gattung Candida sind dabei sowohl ungereinigte Mikroorganismussuspensionen der Gattung Candida als auch gereinigte Enzymfraktionen zu verstehen. Spezies der Gattung Candida (C.) sind beispielsweise C.antarctica, C.rugosa, C.cylindracea. Bevorzugt wird eine Lipase aus C.cylindracea eingesetzt. Lipasen der Gattung Candida sind in verschiedenen Reinheitsstufen käuflich zu erwerben.

Zur Immobilisierung der Lipase wird ein epoxid-aktivierter, makroporöser Träger eingesetzt. Seine Herstellung erfolgt beispielsweise durch Suspensionspolymerisation von Vinylacetat und einem Monomer, das mit Vinylacetat copolymerisierbar ist, z.B. N,N'-Divinylethylenharnstoff in Wasser, partielle Verseifung der Acetat- zu Hydroxygruppen und anschließende Umsetzung mit Epichlorhydrin, das mit freien Hydroxygruppen unter Erhalt des Epoxidringes reagiert. Dabei werden im Polymerisat Spacergruppen mit reaktiven Epoxidgruppen ausgebildet, die zum Verknüpfen des Trägers mit verschiedensten Substraten geeignet sind. Solche Träger und deren Herstellung sind z.B. in K.Burg et al, Angew. Makromol. Chem. 157, (1988), Seiten 105 bis 121 beschrieben. Sie sind käuflich erhältlich. Besonders bevorzugt werden als Träger Eupergit C (Röhm Pharma, Deutschland) oder VA-Epoxy-Biosynth, Riedel de Haen (Deutschland), verwendet.

In A.N.Glazer, "The proteins", ed. H.Neurath und R.L. Hill, Academic press London, 1976, Band II, Seiten 1-109, ist geoffenbart, daß bei dieser Art der Immobilisierung von Enzymen in erster Linie epsilon-Aminogruppen des Lysins mit Epoxidgruppen des Trägers reagieren, wobei Aminogruppen des Lysins alkyliert werden.

Die erfindungsgemäße Lipase ist also an epsilon-Aminogruppen des Lysins der Lipase über geöffente Epoxidgruppen des Trägers alkyliert, d.h. kovalent mit dem Träger verbunden.

Gegenstand der Erfindung ist auch ein Verfahren zur Herstellung der erfindungsgemäßen immobilisierten Lipase. Dazu wird ein epoxid-aktivierter makroporöser Träger mit einer wäßrigen Enzymlösung einer Candida-Lipase bei Temperaturen von 15°C bis zur Desaktivierungstemperatur der Lipase, bevorzugt von 18 bis 30 °C in Kontakt gebracht, beispielsweise etwa in einem Schüttelkolben bewegt. Das Verhältnis von Träger und Lipase muß zumindest so beschaffen sein, daß in der Reaktionsmischung pro freie Aminogruppe des Lysins in der Lipase eine Epoxidgruppe des Trägers vorliegt. Bevorzugt werden auf 1 g Träger etwa 0,05 bis 0,1 g Lipase verwendet. Die Lipase kann dabei in reinem Wasser, einer Puffer- oder Salzlösung gelöst sein. Dabei reagieren epsilon-Aminogruppen des Lysins in der Lipase mit Epoxidgruppen des Trägers, wobei eine N-Alkylierung, also eine kovalente Bindung zwischen Lipase und Träger ausgebildet wird. Nach beendeter Reaktion wird die immobilisierte Lipase gegebenenfalls nach Zugabe von Salzen, z.B. NaCl in die Reaktionsmischung, abfiltriert und mit Pufferlösung gewaschen. Die immobilisierte Lipase kann im Puffer oder feucht oder auch getrocknet bis zum Einsatz aufbewahrt werden.

Die auf diese Weise hergestellte Lipase wird zur enantioselektiven Veresterung von Enantiomerengemischen chiraler Alkohole durch Umesterung unter Verwendung von Enolestern eingesetzt.

Gegenstand der Erfindung ist daher auch ein Verfahren zur enantioselektiven Veresterung eines Alkohols, der mindestens ein Asymmetriezentrum im Molekül enthält, das dadurch gekennzeichnet ist, daß der Alkohol in Gegenwart eines Enolesters und einer Lipase aus einem Mikroorganismus der Gattung Candida, die an epsilon-Aminogruppen des Lysins in der Lipase mit geöffneten Epoxidgruppen eines epoxid-aktivierten, makroskopischen Trägers durch N-Alkylierung kovalent verbunden ist, verestert wird, worauf der gebildete Ester des chiralen Alkohols und gegebenenfalls der nicht umgesetzte Alkohol aus dem Reaktionsgemisch isoliert werden.

Der eingesetzte Alkohol besitzt mindestens ein Asymmetriezentrum und ist daher optisch aktiv. Er kann in Form von racemischen Enantiomerengemischen, oder solchen, in denen das eine oder andere Enantiomer angereichert ist, vorliegen.

Von der erfindungsgemäß immobilisierten Lipase können sowohl primäre, als auch sekundäre Alkohole umgesetzt werden. Der Alkohol kann auch ein Dialkohol mit zwei Asymmetriezentren im Molekül sein, wobei Gemische aus R,S-, S,R-, R,R- oder S,S-Alkohol vorliegen können. Bevorzugt werden sekundäre Alkohole mit einem Asymmetriezentrum im Molekül als Substrat eingesetzt.

Als Enolester können beispielsweise in EP-A-0 321 918 geoffenbarte Enolester eingesetzt werden. Bevorzugt werden Vinylester niedriger organischen Säuren, besonders bevorzugt Vinylacetat, Vinylpropionat, Vinylbutyrat eingesetzt.

Zur Durchführung der Reaktion werden pro Gramm des Enantiomerengemisches des chiralen Alkohols 2 bis 30 g, bevorzugt 6 bis 10 g immobilisierte Lipase und zumindest 5 Äquivalente Enolester, gegebenenfalls mit einem unter den Reaktionsbedingungen inerten Verdünnungsmittel vermischt und unter Rühren oder Schütteln bei Temperaturen von 15 °C bis zur Desaktivierungstemperatur der Lipase, bevorzugt bei Raumtemperatur reagieren gelassen.

Die Reaktion kann in unter den Reaktionsbedingungen inerten Verdünnungsmittel durchgeführt werden oder der zur Umesterung verwendeten Enolester wird selbst auch als Verdünnungsmittel und in hohem Überschuß eingesetzt. Bevorzugt wird die Reaktion nicht in einem unter den Reaktionsbedingungen inerten Verdünnungsmittel sondern in dem zur Umsetzung verwendeten Enolester durchgeführt.

Die Reaktion wird zweckmäßigerweise bei Temperaturen ausgeführt, bei denen die Lipase ihre höchste Aktivität zeigt. Diese Temperatur ist vom Anbieter angegeben oder durch einfach Versuche festzustellen. Dabei bildet sich je nach verwendetem Akoholgemisch, Enolester und Spezifität der eingesetzten Lipase vorwiegend der R- oder vorwiegend der S- bzw. vorwiegend der R,S- oder vorwiegend der S,R-Ester des eingesetzten Alkohols, während der entsprechende S- oder R- bzw. S,R-, R,S-, R,R- oder S,S-Alkohol nicht oder nur zu einem kleinen Anteil umgesetzt wird.

Der Fortgang der Reaktion wird dabei durch geeignete und in der Enzymchemie bekannte Methoden, beispielsweise mit Hilfe gaschromatographischer Methoden, verfolgt.

Die Reaktion wird nach Erreichen der gewünschten Enantiomerenreinheit des Produktes abgebrochen und das Reaktionsgemisch aufgearbeitet. Dazu wird die immobilisierte Lipase abfiltriert und die Mutterlauge einer geeigneten Trennoperation unterzogen, in deren Verlauf die gewünschten Produkte isoliert werden. Die Abtrennung der gewünschten Produkte aus dem Reaktionsgemisch kann dabei etwa mit Hilfe von Extraktion, Destillation, Chromatographie wie üblich erfolgen.

In einer bevorzugten Ausführungsform wird ein racemisches Gemisch eines Alkohols mit Candida cylindracea Lipase, immobilisiert mit Hilfe von VA-Epoxy-Biosynth, Riedel-de-Haen, Deutschland, in Vinylacetat bei Raumtemperatur gerührt oder geschüttelt. Nach Erreichen eines gewünschten Enantiomerenüberschusses des gebildeten R- oder S- bzw. R,S- oder S,R-Esters wird das Enzym abfiltriert und die gewünschten Produkte destillativ oder chromatographisch aufgetrennt.

Es hat sich herausgestellt, daß bei der erfindungsgemäßen Reaktion im Vergleich zu einer nicht immobilisierten Lipase sowohl die Beständigkeit der Lipase gegen Acetaldehyd, als auch ihre Aktivität und Selektivität anstieg. Besonders überraschend war der mehr als 5-fache Anstieg der Selektivität, die beim wiederholten Einsatz der immobilisierten Lipase völlig konstant blieb.

Die immobilisierte Lipase und ihre Verwendung bei der erfindungsgemäßen enantioselektiven Veresterung stellen daher eine Bereicherung der Technik dar.

**Beispiel 1**

10,0 g VA-Epoxy-Biosynth (Riedel-de-Haen, Deutschland) wurden in 70 ml 0,1 n PHosphatpuffer, pH 7,00, suspendiert und nach Zugabe von 300 mg Lipase von Candida cylindracea Lipase (AY-30, Firma Amano Pharm. Co, Japan) bei 27 °C und 80 U.p.min 3 Tage geschüttelt. Nach Zugabe von 70 ml Natriumchloridlösung wurde abfiltriert, zweimal mit je 30 ml 0,05 n Phosphatpuffer, pH 7 gewaschen und getrocknet.

Die spezifische Aktivität der derart hergestellten immobilisierten Lipase wurde durch Titration der gebildeten Essigsäure mit 0,1 n Natronlauge während der Hydrolyse von Triacetin in 0,1 n Phosphatpuffer, pH 7,00, bestimmt und betrug 5,70 $\mu$mol min$^{-1}$ g$^{-1}$. Die spezifische Aktivität der nicht immobilisierten Lipase betrug unter den gleichen Bedingungen 13 $\mu$mol min$^{-1}$ g$^{-1}$.

**Beispiel 2**

1 g racemisches endo-Norborn-5-en-2-ol (9 mmol) wurde mit 200 mg Candida cylindracea Lipase (Ay-30, Firma Amano Pharm. Co., Japan) vermischt und in 10 ml Vinylacetat bei 20 °C und 200 U.p.min geschüttelt. Der Fortgang der Reaktion wurde gaschromatographisch verfolgt. Nach 4 Stunden wurde die Reaktion abgebrochen. Die Lipase wurde abfiltriert und die Mutterlauge im Vakuum eingedampft. Nach konventioneller Säulenchromatographie des Rückstandes über Kieselgel wurden ( + )-endo-Norborn-5-en-2-yl Acetat und (-)-endo-Norborn-5-en-2-ol erhalten.
Die Untersuchungsergebnisse betreffend Lipaseaktivität, Umsatz, Enantiomerenverhältnisse sind in Tabelle 1 zusammengefaßt.

**Beispiel 3**

wurde wie Beispiel 2 durchgeführt, mit dem Unterschied, daß die nach Beispiel 1 immobilisierte Lipase eingesetzt wurde. Die Untersuchungsergebnisse betreffend Lipaseaktivität, Umsatz, Enantiomerenverhältnisse sind in Tabelle 1 zusammengefaßt.

## Tabelle 1

| A | Lipase | Akt. | % ee (-) Alk. | % ee (+)Est | S |
|---|--------|------|---------------|-------------|---|
| 1. | V | 39 | 51,4 | 66,5 | 8 |
| 2. | V | 1 | 11,0 | 54,0 | 4 |
| 3. | V | - | - | - | - |
| 1. | I | 100 | 62,2 | 91,3 | 42 |
| 2. | I | 50 | 66,0 | 91,7 | 42 |
| 3. | I | 23 | 59,1 | 91,8 | 42 |

In der Tabelle bedeuten

A: Anzahl der Verwendungen

V Vergleich

I: immobilisierte Lipase

Akt: Relative Aktivität, bestimmt durch Vergleich des Anstieges des Reaktionsverlaufes über die ersten 20 % der Reaktion (Tangentenmethode)

%ee (-)Alk: Enantiomerenüberschuß des nicht umgesetzten (-)-endo-Norborn-5-en-2ol

%ee (+)Est: Enantiomerenüberschuß des gebildeten (+)-endo-Norborn-5-en-2-yl-acetat

Der jeweilige Enantiomerenüberschuß wurde mit Hilfe gaschromatographischer Auftrennung der entsprechenden Menthylchlorformate, die durch Derivatisierung mit (-)-Menthylchlorformat nach B. Berger et al, Tetrahedron: Asymmetry, 1 (1990), Seiten 541-546, hergestellt wurden, bestimmt.

S: Enantioselektivität der Reaktion

Bestimmt nach der Methode von Chen et al., J.Am.Chem. Soc, 104 (1982), Seiten 7294-7299

**Patentansprüche**

1. Lipase aus einem Mikroorganismus der Gattung Candida, dadurch gekennzeichnet, daß epsilon-Aminogruppen des Lysins in der Lipase durch N-Alkylierung kovalent über geöffnete Epoxidgruppen eines epoxid-aktivierten makroporösen Trägers gebunden sind, wodurch die Lipase immobilisiert wird.

2. Lipase nach Anspruch 1, dadurch gekennzeichnet, daß der makroskopische Träger durch Suspensionspolymerisation von Vinylacetat und einem Monomer, das mit Vinylacetat copolymerisierbar ist, in Wasser, partielle Verseifung der Acetat zu Hydroxygruppen und anschließende Umsetzung mit Epichlorhydrin, das mit freien Hydroxygruppen unter Erhalt des Epoxidrings reagiert, hergestellt wird.

3. Verfahren zur Immobilisierung einer Lipase aus einem Mikroorganismus der Gattung Candida, dadurch gekennzeichnet, daß ein epoxid-aktivierter makroporöser Träger mit einer Lösung einer Candida-Lipase in Wasser, einer Puffer- oder einer Salzlösung bei Temperaturen von 15 °C bis zur Desaktivierungstemperatur der Lipase umgesetzt wird, wobei durch N-Alkylierung kovalente Bindungen zwischen epsilon-Aminogruppen des Lysins in der Lipase mit geöffneten Epoxidgruppen des epoxid-aktivierten makroporösen Trägers ausgebildet werden.

4. Verfahren nach Anspruch 3, dadurch gekennzeichnet, daß pro g des makroporösen Trägers 0,05 bis 0,1 g Lipase eingesetzt werden.

5. Verwendung einer Lipase nach Anspruch 1 zur enantioselektiven Veresterung eines chiralen Alkohols mit Hilfes eines Enolesters.

6. Verfahren zur enantioselektiven Veresterung eines chiralen Alkohols, dadurch gekennzeichnet, daß der Alkohol in Gegenwart eines Enolesters und einer Lipase aus einem Mikroorganismus der Gattung Candida, die durch N-Alkylierung über epsilon-Aminogruppen des Lysins mit Epoxidgruppen eines epoxid-aktivierten makroporösen Trägers kovalent verbunden ist, verestert wird, worauf der gebildete Ester des chiralen Alkohols und gegebenenfalls der nicht umgesetzte Alkohol aus dem Reaktionsgemisch isoliert werden.

7. Verfahren nach Anspruch 6, dadurch gekennzeichnet, daß auf ein Gramm des chiralen Alkohols mindestens 5 Äquivalente Enolester und 2 bis 30 Gramm immobilisierte Lipase eingesetzt werden.

8. Verfahren nach einem der Ansprüche 6 oder 7, dadurch gekennzeichnet, daß als Verdünnungsmittel für die Veresterung jener Enolester, der auch als Reaktionspartner verwendet wird, eingesetzt wird.

9. Verfahren nach einem der Ansprüche 6 bis 8, dadurch gekennzeichnet, daß als Enolester Vinylacetat, Vinylpropionat oder Vinylbutyrat eingesetzt wird.

10. Verfahren nach einem der Ansprüche 6 bis 9, dadurch gekennzeichnet, daß als Lipase eine Lipase aus einem Mikroorganismus der Spezies Candida cylindracea eingesetzt wird.